# EUROPEAN PATENT APPLICATION

(11) **EP 2 591 825 A1**
(43) Date of publication of application: **15.05.2013**
(21) Application number: 11188201.5
(22) Date of filing: 08.11.2011
(51) Int. Cl.: A61Q 11/00, A61K 8/24, A61K 8/34, A61K 8/60

(54) **Composition for the treatment of teeth, and use**

(71) Applicant: PeGoMa B.V., 1079 PB Amsterdam (NL)
(72) Inventor: Cuppen, Martinus Josephus Maria, 1079PB Amsterdam (NL); Spaid, Matthew Scott, 1079PB Amsterdam (NL)
(74) Representative: Jansen, Bart Antonius Johannes

(57) **Abstract**

The invention relates to a composition for the treatment of teeth, comprising hydroxyapatite nanoparticles and at least one antibacterial polyol, and, optionally, other components. The composition may be used in the treatment of teeth, for maintenance or repair of the tooth enamel. The composition may for instance be a dentifrice, an active tooth foam, a mouth rinse, a swab, gel treatment, a spray, a wipe, a prophylactic paste, a post-whitening repair crème, a chewing gum, effervescent tablets and a tooth gloss.

## Description

### FIELD OF THE INVENTION

The invention relates to a composition for the treatment of teeth, and a use thereof.

### BACKGROUND OF THE INVENTION

Hydroxyapatite (also known as hydroxyl apatite) is a naturally occurring mineral form of calcium apatite with the general formula Ca₅(PO₄)₃(OH). This mineral is a major component of teeth, in particular the dental enamel and dentin parts.

The use of hydroxyapatite nanoparticles for the treatment of human teeth for maintaining and repairing damaged teeth surface has been investigated. It is postulated that hydroxyapatite nanoparticles fill the holes, scratches and other damaged parts of the teeth surface, in particular repairing the enamel.

The European patent application EP1098623 "ODONTOSTOMATOLOGIC USE OF APATITE-BASED NANOSTRUCTURED MATERIALS" discusses the use of apatite nanomaterials (sizes 0.5-200 nm diameter) for dental applications.

WO 2005020878 "ORAL AND DENTAL CARE PRODUCT" describes the use of calcium phosphate particles (including hydroxyapatite) in tooth paste, with a nanoparticle length from 5-150 nm and a diameter of 2-50 nm. Hydroxyapatite nanoparticles are a relatively expensive ingredient. Also, formulations using hydroxyapatite nanoparticles may sometimes cause unpleasant sensations in the mouth of the user.

### OBJECT AND SUMMARY OF THE INVENTION

It is an object of the invention to provide a new and improved hydroxyapatite nanoparticles formulation.

The invention provides a composition for the treatment of teeth, comprising hydroxyapatite nanoparticles and at least one antibacterial polyol, and, optionally, other components. Surprisingly, combining hydroxyapatite nanoparticles and antibacterial polyols has a synergetic effect in protecting teeth. It is postulated that the combination of the polyol effectively killing bacteria on the tooth surface and the hydroxyapatite particles filling up small holes and crevices, may cause the superior effectiveness. The treatment of with the combination of hydroxyapatite nanoparticles and a polyol also resulted in a smoother tooth surface. The combination also showed an improved result in the treatment as well as prevention of gingivitis. Also, the combination leads to a more lustrous tooth surface, giving it a more reflective and whiter appearance.

Hydroxyapatite nanoparticles may be obtained commercially, and typically have an average particle size below 1 µm, as measured by conventional methods such as those mentioned in EP1098623 and references therein. Hydroxyapatite also includes partially modified hydroxyapatite, for instance wherein part of the hydroxyl groups in the material are substituted by fluoride or chloride. It is postulated that the smaller the size of the hydroxyapatite nanoparticles works better than larger sizes because the smaller particles are able to fit in smaller damaged areas of the enamel

In addition, a source of fluorine ions could be added. Fluorine is known to increase the protection of teeth by forming a more resistant fluoroapatite layer in reaction with hydroxyapatite, which is the major constituent of tooth enamel. Suitable fluoride compounds include sodium fluoride, potassium fluoride, disodium monofluorophosphate, dipotassium monofluorophosphate, and stannous fluorophosphates as well as several organic fluor compounds known for use in tooth paste and other compositions for the treatment of teeth.

Additional ingredients could include thickeners such as water soluble natural or synthetic polymers such as alginates, carrageenan, agar, guar gum, arabic gum, xanthan, pectin, cellulose and derivatives thereof, starch and modified starch. Other possibilities include carboxyvinylpolymers, carbopol, polyethylene glycol, and glycerine. As fillers, inorganic materials such as titanium dioxide, silicium aluminium oxides and silicium oxides may be used.

Preferably, the composition has an alkaline pH above 7, preferably in the range of from 8-8.5. Acids, bases and/or pH buffers may be added to achieve the desired pH.

Further functional ingredients include additional anti-caries compounds, in particular, antimicrobial compounds, dental stone inhibitors and taste masking ingredients such as peppermint and spearmint oil.

It is preferred if the composition is selected from the group consisting of a dentifrice, an active tooth foam, a mouth rinse, a swab, gel treatment, a spray, a wipe, a prophylactic paste, a post-whitening repair crème, a gum, effervescent tablets and a tooth gloss. These compositions differ in their viscosity and appearance, as well as in their way of applying to teeth in the mouth. Foam is particularly advantageous, as this allows the efficient covering of the tooth surface, using a relatively low amount of active ingredients, and is able to penetrate interproximal surfaces of the teeth.

In a preferred embodiment, the hydroxyapatite nanoparticles have an average diameter of from 1 to 200 nm. It is believed particles in this range show a particularly good synergistic effect with polyols. Particle sizes may be determined with methods known in the art, for instance the methods described in WO 2005020878, included by reference. The best effects are believed to be achieved when the hydroxyapatite particles have an average diameter of from 5-10 nm.

It is preferred if the amount of hydroxyapatite nanoparticles is at least 1% w/w of the composition, preferably at least 5% w/w, more preferably at least 10% w/w. Such an amount is preferred if a rapid enamel repair rate is wanted. Relatively low amounts of nanoparticles would need a longer exposure of the teeth surface to the composition.

Preferably, the polyol is selected from the group consisting of maltitol, lactitol, erythritol, xylitol, polyglycitol and isomalt. These polyols are easily available, considered to be safe when (accidentally) ingested by a user of compositions as described herein, and show a good antibacterial action and synergistic effect with hydroxyapatite nanoparticles. Combinations of antibacterial polyols may also be used. Best synergetic anti-caries-results results are obtained if the polyol is xylitol or erythritol.

It is advantageous if the composition comprises at least 0.1 % w/w polyol, in particular xylitol or erythritol. For polyols with a lower antibacterial effect than xylitol, higher minimum amounts may be preferred. More preferably, the composition comprises at least 1% of polyol. At such concentrations, the polyol provides a masking effect for the taste and texture of the hydroxyapatite nanoparticles. Xylitol and erythrytol were found to be particularly effective in masking.

It is advantageous if the composition also comprises a nerve numbing agent. Adding the nerve numbing agent reduces the sensitivity of the teeth and surrounding tissue. The combination of hydroxyapatite nanoparticles, at least one antibacterial polyol (preferably xylitol) and a nerve numbing agent showed excellent results in treating and preventing gingivitis, in particular in elderly persons. It is preferred if the mind numbing agent comprises a nitrate salt, in particular potassium nitrate or sodium nitrate.

Preferably, the composition the composition also comprises at least 1% w/w of smectite clay. Smectite clay was found to be an excellent agent to stabilize and suspend hydroxyapatite nanoparticles in the presence of a polyol, in particular xylitol. The smectite clay is particularly suitable for stabilizing tooth paste and similar viscous formulations. A popular type of smectite clay that worked well is sold under the trade name Veegum D. In a preferred embodiment, the composition comprises from 1 to 3% w/w smectite clay. Good stability may be obtained if the composition comprises smectite clay to hydroxyapatite nanoparticles in a weight ratio of from 1:5 to 5:1.

It is preferred if the composition also comprises at least one surfactant, preferably an anionic surfactant. Having a surfactant is in particular important for a homogenous mixture, in particular in forming foam formulations. Depending on other ingredients, the optimal range is typically from at least 0.1 % w/w to 3% w/.w. Suitable anionic surfactants include sodium dodecyl sulfate, sodium sarcosinate, or their respective potassium or quartenary ammonium salts, sodium laureth sulphate, water-soluble salts of higher fatty acid monoglyceride monosulfates, such as the sodium salt of the monosulfated monoglyceride, or hydrogenated coconut oil fatty acids such as sodium N-methyl N-cocoyl taurate, sodium cocomo-glyceride sulfate, higher alkyl sulfates such as sodium lauryl sulfate, alkyl aryl sulfonates such as sodium dodecyl benzene sulfonate, higher alkyl sulfoacetates, such as sodium lauryl sulfoacetate (dodecyl sodium sulfoacetate), higher fatty acid esters of 1,2 dihydroxy propane sulfonate, sulfocolaurate (N-2-ethyl laurate potassium sulfoacetamide) and sodium lauryl sarcosinate. Another class of surfactants that were found suitable are cocamidepropyl-derived surfactants, in particular cocamide diethanolamine and cocamidopropyl betaine. Sarcosinate salts, in particular sodium sarcosinate or potassium sarcosinate, showed a particularly good compatibility with hydroxyapatite nanoparticles in combination with antibacterial polyols.

Compositions may be packaged in various dispensers and containers, including a tube, ampoule, spray, swab or wipe.

The invention also provides the use of a composition as described herein for the treatment of teeth. The treatment may for instance be aimed at maintenance or repair of the tooth enamel. The treatment may be carried out by daily domestic use of the compositions as described herein, but may all so be carried out at professional dental care centres by dentists and dental hygienists.

### DESCRIPTION OF PREFERRED EMBODIMENTS

The invention will now be further elucidated by the following non-limiting examples. The ingredients used are commercially available, and were mixed using mixing techniques known in the art.

The hydroxyapatite paste used in preparation examples below contains approximately 15% w/w of dry weight in nanoparticles with an average diameter of 5-10 nm, suspended in an aqueous mixture. The hydroxyapatite nanoparticles showed a good effect for teeth enamel remineralisation when combined with the other ingredients, decreased dentin hypersensitivity and enhanced teeth whitening and gloss.

Smectite clay used in the example is sold under the trade name 'Veegum D'.

Sodium sarcosinate is an anionic surfactant sold under the trade name Crodasinic LS30

### Example 1: Toothpaste

| **Ingredient** | **% w/w** |
|---|---|
| Hydroxyapatite paste 15% | 15 |
| Xylitol | 10 |
| Potassium nitrate | 5 |
| Smectite clay | 1.5 |
| Sorbitol | 20 |
| Glycerine | 10 |
| Sodium sarcosinate | 3 |
| Silicium oxide | 15 |
| Titanium dioxide | 1 |
| Xanthan gum | 1 |
| Peppermint oil | 1 |
| Demineralised water | up to 100% |

Users of this toothpaste indicated a smoother and more lustrous tooth surface than obtained with previously known tooth pastes. In an alternative formulation, 12% erythritol was used instead of xylitol.

The toothpaste composition is prepared in a high shear mixer while heating, in order to obtain a good distribution of the hydroxyapatite nanoparticles and polyol.

### Example 2: Tooth foam

This formulation has a foamy structure and lower viscosity than the toothpaste, allowing for a more rapid application of the active ingredients on the tooth surface.

| **Component** | **% w/w** |
|---|---|
| Hydroxyapatite paste 15% | 15 |
| Xylitol | 7 |
| Potassium nitrate | 3 |
| Smectite clay | 2 |
| Sodium sarcosinate | 4 |
| Phenoxyethanol | 1 |
| Peppermint oil | 1 |
| Demineralised water | up to 100% |

In an alternative formulation, instead of 7% w/w xylitol, a mixture of 4% xylitol and 4% erythritol was used.

### Example 3: Mouth rinse

This product is essentially liquid to allow for a thorough rinsing of the mouth.

| **Ingredient** | **% w/w** |
|---|---|
| Hydroxyapatite paste15% | 2 |
| Xylitol | 5 |
| Potassium nitrate | 1 |
| Glycerine | 5 |
| Sodium sarcosinate | 1.5 |
| Polyoxyethylene 40 | 2 |
| Peppermint oil | 1 |
| Demineralised water | up to 100% |

### Example 4: Chewing gum

This product is essentially solid, and can be chewed to give a cleaning and repair of the tooth surface.

Gum base 30.00%
Nano-Hydroxyapetite 15%
Xylitol 43.80%
Peppermint powder 0.50%
Menthol powder 0.20%
Magnesium stearate 0.50%

The gum base may be a known gum base for chewing gums, for instance be Alternatively, a lozenge may be made, using a lozenge base formulation instead of a gum base formulation.

## Claims

1. Composition for the treatment of teeth, comprising hydroxyapatite nanoparticles and at least one antibacterial polyol, and, optionally, other components.

2. Composition according to claim 1, wherein the composition is selected from the group consisting of a dentifrice, an active tooth foam, a mouth rinse, a swab, gel treatment, a spray, a wipe, a prophylactic paste, a post-whitening repair crème, a gum, effervescent tablets and a tooth gloss.

3. Composition according to claim 1 or 2, wherein the hydroxyapatite particles have an average diameter of from 1-200 nm, preferably from 5-10 nm.

4. Composition according to any of the preceding claims, wherein the amount of hydroxyapatite nanoparticles is at least 1% w/w of the composition, preferably at least 5% w/w, more preferably at least 10% w/w.

5. Composition according to any of the preceding claims, wherein the polyol is selected from the group consisting of maltitol, lactitol, erythritol, xylitol, polyglycitol and isomalt.

6. Composition according to claim 6 wherein the polyol comprises xylitol and/or erythritol.

7. Composition according to any of the preceding claims wherein the composition comprises at least 0.1% w/w polyol, in particular xylitol and/or erythritol.

8. Composition according to any of the preceding claims wherein the composition comprises hydroxyapatite nanoparticles and polyols in a weight ratio of from 1 to 10 to 10:1.

9. Composition according to any of the preceding claims, wherein the composition also comprises a nerve numbing agent.

10. Composition according to claim 9, wherein the mind numbing agent comprises a nitrate salt, in particular potassium nitrate or sodium nitrate

11. Composition according to any of the proceeding claims, wherein the composition also comprises at least 1% w/w of smectite clay.

12. Composition according to any of the preceding claims, wherein the composition also comprises at least one at least one surfactant, preferably an anionic surfactant.

13. Composition according to claim 12, wherein the anionic surfactant comprises a sarcosinate salt, in particular sodium sarcosinate or potassium sarcosinate.

14. Composition according to any of the preceding claims, wherein the composition has an alkaline pH above 7, preferably in the range of from 8-8.5.

15. Use of a composition according to any of the preceding claims for the treatment of teeth.
